# EUROPEAN PATENT SPECIFICATION

(11) **EP 3 648 820 B1**
(45) Date of publication and mention of the grant of the patent: **29.11.2023**
(21) Application number: 18827348.6
(22) Date of filing: 04.07.2018
(51) Int. Cl.: A61M 15/00

(54) **DILUTION SPACER AND ACTUATOR FOR METERED-DOSE INHALER**
VERDÜNNUNGSABSTANDSHALTER UND AKTUATOR FÜR DOSIERINHALATOR
ESPACEUR DE DILUTION ET ACTIONNEUR POUR INHALATEUR-DOSEUR

(30) Priority: 07.07.2017 US 201715644641
(43) Date of publication of application: 13.05.2020
(73) Proprietor: Andrus, Paul G., Ancaster, Ontario L9K 1M5 (CA); Campbell-Andrus, Gayle R., Ancaster, Ontario L9K 1M5 (CA)
(72) Inventor: Andrus, Paul G., Ancaster, Ontario L9K 1M5 (CA); Campbell-Andrus, Gayle R., Ancaster, Ontario L9K 1M5 (CA)
(74) Representative: Patentanwälte Olbricht Buchhold Keulertz
(86) International application number: PCT/CA2018/050814
(87) International publication number: WO 2019/006547

(56) References cited:
- EP-A1- 0 009 667
- WO-A1-2010/076683
- WO-A1-2010/076683
- WO-A1-2014/143173
- WO-A1-2014/143173
- US-A- 4 534 343
- US-A- 4 852 561
- US-A- 4 852 561
- US-A- 5 042 467
- US-A- 5 042 467
- US-A1- 2012 247 460
- US-A1- 2016 367 770
- US-B1- 6 681 767
- US-B1- 7 418 962

## Description

### TECHNICAL FIELD

The present disclosure relates to metered-dose inhalers, and more particularly to dilution spacers for use with metered-dose inhalers.

### BACKGROUND

A metered-dose inhaler (MDI) is a device that delivers a measured quantity of aerosolized medication.

Dilution spacers are known from the prior art, e.g. from WO 2010/076683 A1, US 5042467 A, US 2016/367770 A1, US 6684767 B1, US 4852561 A or EP 0009667 A1.

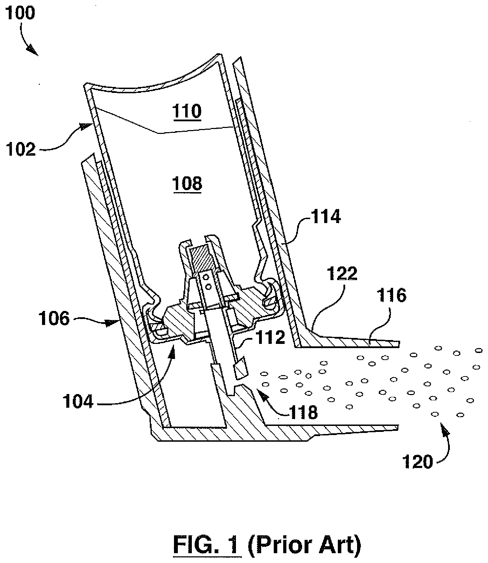

Referring now to Figure 1, one illustrative example of a metered-dose inhaler, indicated generally by reference 100, will be described. The metered-dose inhaler 100 of Figure 1 is made up of three primary components: a canister 102, a metering valve 104 and an actuator 106. The canister 102 is typically formed from stainless steel or aluminum, and contains the material to be dispensed; i.e. the medication 108 and propellant 110 (other materials such as excipients may also be included). The metering valve 104 is sealed to the canister 102, and includes a movable hollow valve stem 112. The metering valve 104 is configured so that when the valve stem 112 is moved toward the canister 102 from a containing position into a dispensing position (i.e. when the valve is actuated), a metered quantity of the medication 108 is released from the canister 102 through the valve stem 112. The configuration of the metering valve 104 is such that even if the valve stem 112 is maintained in the dispensing position, only the metered quantity of medication 108 is dispensed. Design and construction of metering valves is well known, and is not discussed further. The actuator 106 comprises a hollow body 114 that receives the canister 102, a mouthpiece 116, typically projecting obliquely from the body 114, and an actuator nozzle 118, also referred to as an atomizing nozzle, projecting inwardly at the junction of the body 114 and the mouthpiece 116. The valve stem 112 is received by the actuator nozzle 118 in fluid communication therewith so that pushing the canister 102 toward the actuator nozzle 118 moves the valve stem 112 (relative to the canister 102) into the dispensing position and releases the metered quantity of medication 108 into the actuator nozzle 118. The actuator nozzle 118 is configured to generate a plume 120 from the contents of the metered-dose inhaler canister 102 received through the valve stem 112 and direct the plume 120 through the mouthpiece 116. A patient would administer a dose of the medication 108 by pressing the canister 102 into the body 114 of the actuator 104 while inhaling through the mouthpiece 106.

Inhaling directly from a metered-dose inhaler can be difficult, and patients may use a tube having a mouthpiece at one end and a receptacle that receives the actuator mouthpiece 116 at the other end. These tubes, referred to as holding chambers or spacers, function as a reservoir to contain the metered dose inhaler plume 120, making it easier to inhale. However, such holding chambers or spacers are generally large and cumbersome.

### SUMMARY

It is disclosed a dilution spacer for a metered-dose inhaler comprising an enclosure defining a dilution chamber, an actuator inlet configured to securely releasably interengage a metered-dose inhaler actuator mouthpiece, an ambient air inlet and an outlet. Each of the actuator inlet, the ambient air inlet and the outlet are in fluid communication with the dilution chamber. The ambient air inlet is positioned opposite the outlet whereby suction through the outlet from outside the enclosure draws ambient air into the enclosure through the ambient air inlet to generate an airflow path from the ambient air inlet through the dilution chamber and out of the outlet. The actuator inlet is positioned relative to the ambient air inlet and the outlet so that a metered-dose inhaler plume entering the dilution chamber through the actuator inlet intersects the airflow path thereto, whereby airflow along the airflow path entrains and redirects at least a portion of the metered-dose inhaler plume toward the outlet.

The dilution spacer may be incorporated into an assembly further comprising a metered-dose inhaler actuator whose actuator mouthpiece is securely releasably interengaged in the actuator inlet. The actuator mouthpiece may, for example, be friction fit in the actuator inlet or be interference fit in the actuator inlet.

The assembly may further comprise a metered-dose inhaler canister received in the body of the metered-dose inhaler actuator, and the valve stem of the metering valve sealed to the metered-dose inhaler canister may be received by the actuator nozzle of the metered-dose inhaler actuator. The actuator nozzle is configured to generate a metered-dose inhaler plume from contents of the metered-dose inhaler canister and direct the metered-dose inhaler plume into the dilution chamber through the actuator mouthpiece and the actuator inlet. In certain preferred embodiments, the actuator nozzle is configured to direct the metered-dose inhaler plume at an oblique angle to the valve stem.

In some embodiments, the enclosure is generally parallelepipedic, the actuator mouthpiece of the metered-dose inhaler actuator is at an oblique angle to the body of the metered-dose inhaler actuator and the body of the metered-dose inhaler actuator is substantially flush with an edge of the enclosure in which the actuator inlet is formed.

In certain preferred embodiments, the metered-dose inhaler plume entering the dilution chamber through the actuator inlet intersects the airflow path non-parallel thereto, and in certain particular embodiments, the airflow redirects at least a portion of the metered-dose inhaler plume by about 103.5 degrees.

In some embodiments, the outlet comprises a dilution spacer mouthpiece projecting outwardly from the enclosure.

It is also disclosed a metered-dose inhaler actuator comprising an enclosure defining a dilution chamber, a receptacle having an actuator nozzle and configured to receive a metered- dose inhaler canister so that the valve stem of the metering valve sealed to the metered-dose inhaler canister is received by the actuator nozzle, and further comprising an ambient air inlet and an outlet. Each of the actuator nozzle, the ambient air inlet and the outlet are in fluid communication with the dilution chamber. The ambient air inlet is positioned opposite the outlet whereby suction through the outlet from outside the enclosure draws ambient air into the enclosure through the ambient air inlet to generate an airflow path from the ambient air inlet through the dilution chamber and out of the outlet. The actuator nozzle is configured to generate a metered-dose inhaler plume from contents of the metered-dose inhaler canister and direct the metered-dose inhaler plume into the dilution chamber. The actuator nozzle is positioned relative to the ambient air inlet and the outlet so that the metered-dose inhaler plume intersects the airflow path, whereby airflow along the airflow path entrains and redirects at least a portion of the metered-dose inhaler plume toward the outlet.

In some embodiments, the outlet comprises a mouthpiece projecting outwardly from the enclosure.

The metered-dose inhaler actuator may be incorporated into an assembly further comprising a metered-dose inhaler canister received within the receptacle with the valve stem of the metering valve of the metered-dose inhaler canister received by the actuator nozzle. In certain preferred embodiments, the actuator nozzle is configured to direct the metered-dose inhaler plume at an oblique angle to the valve stem.

In certain preferred embodiments, the metered-dose inhaler plume entering the dilution chamber through the actuator inlet intersects the airflow path non-parallel thereto, and in certain particular embodiments, the airflow redirects at least a portion of the metered-dose inhaler plume by about 103.5 degrees.

The invention is defined by independent claims 1 and 12. Further aspects are object of the dependent claims.

### BRIEF DESCRIPTION OF THE DRAWINGS

These and other features will become more apparent from the following description in which reference is made to the appended drawings wherein: FIGURE 1 is a side cross-sectional view of an illustrative metered-dose inhaler;
FIGURE 2 is a top front perspective view of an illustrative dilution spacer for a metered-dose inhaler;
FIGURE 3 is a bottom rear perspective view of the dilution spacer of Figure 2; FIGURE 4 is a right side elevation view of the dilution spacer of Figure 2; FIGURE 5 is a front elevation view of the dilution spacer of Figure 2;
FIGURE 6 is a left side elevation view of the dilution spacer of Figure 2; FIGURE 7 is a rear elevation view of the dilution spacer of Figure 2; FIGURE 8 is a top plan view of the dilution spacer of Figure 2; FIGURE 9 is a bottom plan view of the dilution spacer of Figure 2;
FIGURE 10 is a cross-sectional view of the dilution spacer of Figure 2, taken along the line 10-10 in Figure 5;
FIGURES 10A and 10B show cross-sectional views of the dilution spacer of Figure 2, taken along the line 10-10 in Figure 5, in combination with the metered-dose inhaler of Figure 1 ;
FIGURE 11 is a cross-sectional view of an illustrative metered-dose inhaler actuator; and
FIGURES 11 A and 11B show cross-sectional views of the metered-dose inhaler actuator of Figure 11 in combination with a metered-dose inhaler canister and metering valve.

### DETAILED DESCRIPTION

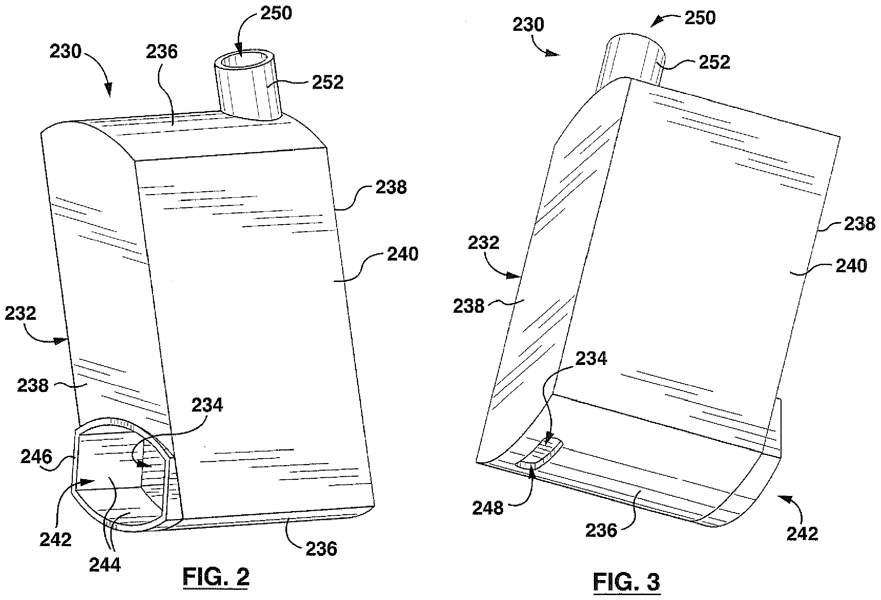

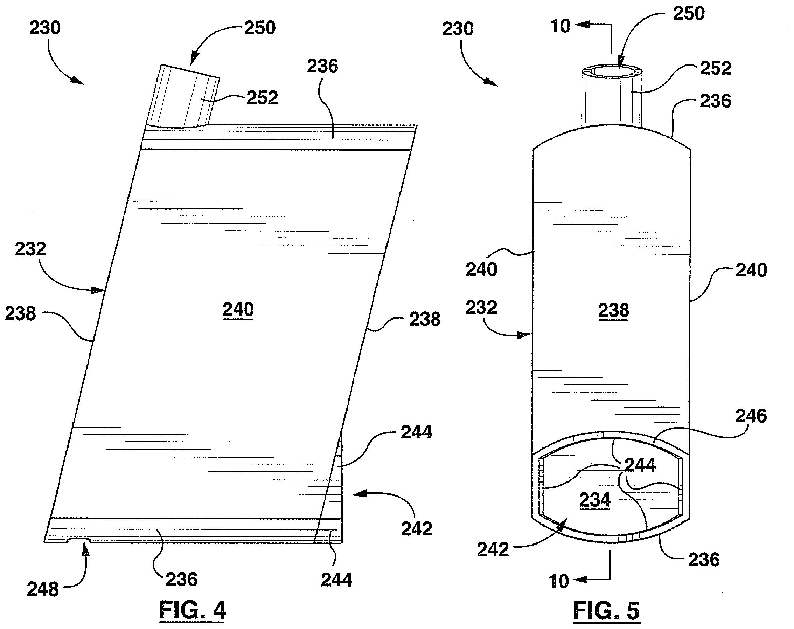

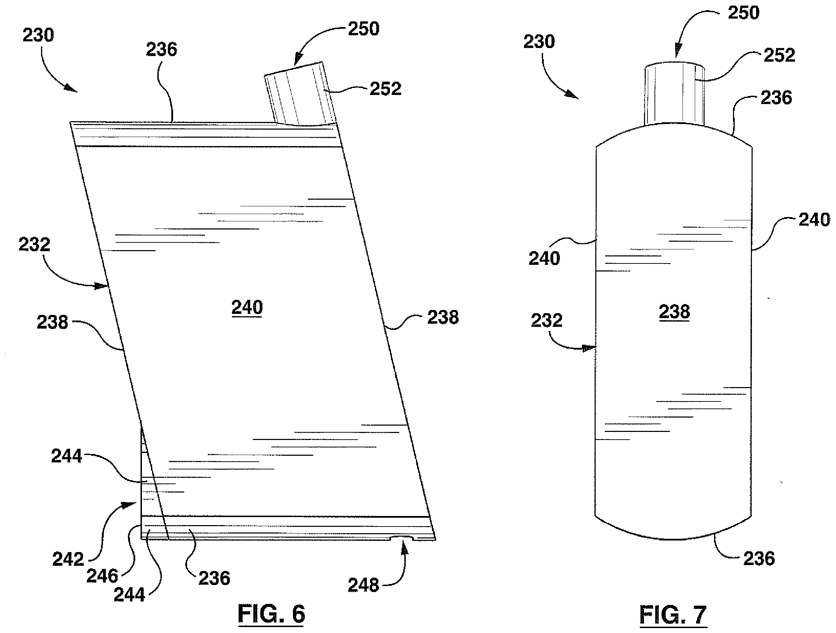

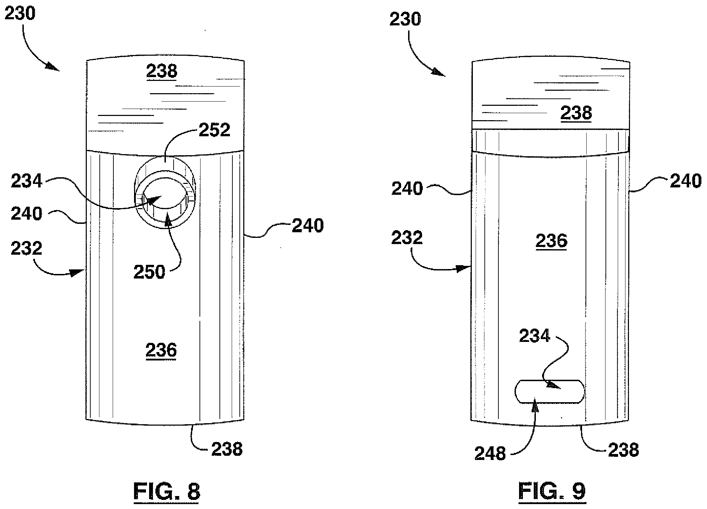

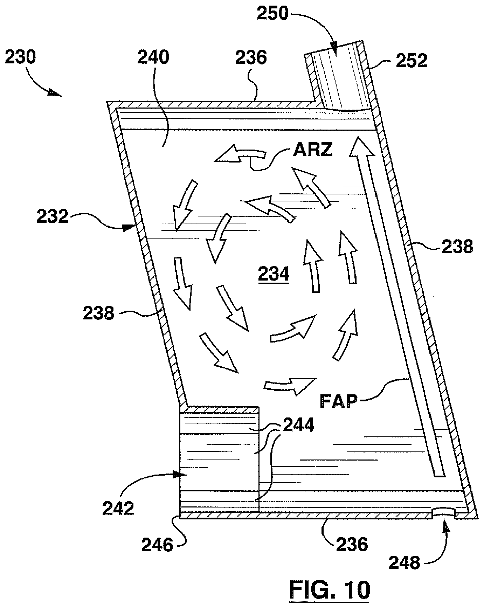

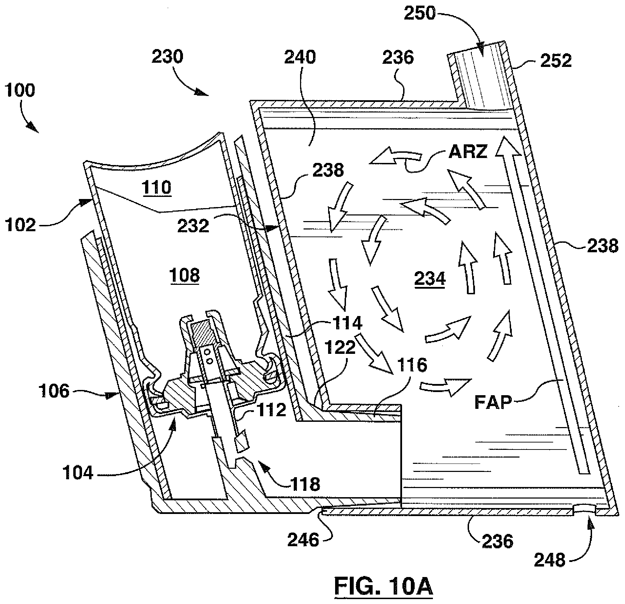

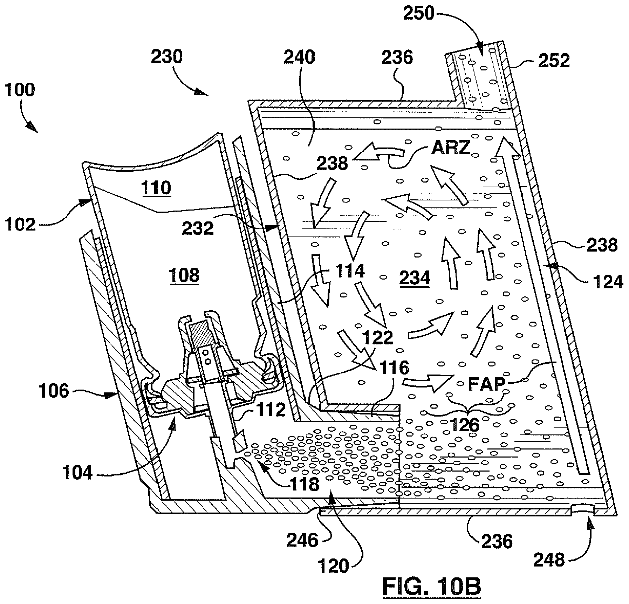

Reference is now made to Figures 2 to 11, with particular emphasis on the cross- sectional views shown in Figures 10 to 10B. In Figures 2 to 11, an illustrative dilution spacer for a metered-dose inhaler is indicated generally by reference 230. The dilution spacer 230 comprises an enclosure 232 defining a dilution chamber 234 (Figures 10 to 10B). In the illustrative embodiment shown in Figures 2 to 10B, the enclosure 232 is generally parallelepipedic and is formed by three pairs of opposed walls. More particularly, the enclosure 232 is formed by a pair of opposed outwardly curved end walls 236, a pair of opposed generally planar edge walls 238 and a pair of opposed generally planar side walls 240. Although the end walls 236 are generally curved, the overall shape of the enclosure is generally parallelepipedic. The shape of the enclosure 232 shown in Figures 2 to 11 is merely illustrative, and a wide range of shapes may be applied to the enclosure without departing from the scope of the present disclosure.

An actuator inlet 242 is formed through one of the edge walls 238, and is in fluid communication with the dilution chamber 234. The actuator inlet 242 is configured to securely releasably interengage an actuator mouthpiece 116, as shown in Figures 10A and 10B, and includes inlet walls 244 (best seen in Figure 10) whose size and shape is complementary to that of the actuator mouthpiece 116 to be received. In the illustrated embodiment, the actuator inlet 242 includes an alignment lip 246. As can be seen in Figures 10A and 10B, the actuator mouthpiece 116 of the actuator 104 is at an oblique angle to the body 114 of the actuator 104 and the alignment lip 246 engages a shoulder 122 on the actuator mouthpiece 116. In the illustrated embodiment, the angle formed between the body 114 of the actuator 104 and the actuator mouthpiece 116 is approximately equal to the angle between the edge wall 238 in which the actuator inlet 242 is formed and the end wall 236 adjacent the actuator inlet 242; the angle will depend on the geometry of the actuator 106 with which the dilution spacer 230 is to be used. As a result, when the actuator mouthpiece 116 is received in the actuator inlet 242, the body 114 of the actuator 104 is substantially flush with the edge wall 238 in which the actuator inlet 242 is formed and therefore is substantially flush with that edge of the enclosure 232; there may be a small gap as shown in the drawings. The actuator mouthpiece 116 may be friction fit in the actuator inlet 242 or interference fit in the actuator inlet, or may be secured by other suitable technique.

An ambient air inlet 248 is formed through one of the end walls 236 and an outlet 250 is formed through the other one of the end walls 236 and positioned opposite the ambient air inlet. In the illustrated embodiment, the outlet 250 comprises a dilution spacer mouthpiece 252 projecting outwardly from the respective end wall 236 and hence outwardly from the enclosure 232; in alternate embodiments the dilution spacer mouthpiece may have a wide range of different shapes or may be omitted and the outlet may consist of a simple aperture through the respective end wall. The ambient air inlet 248 and the outlet 250 are in fluid communication with the dilution chamber 234.

Reference is now made to Figure 10A, which shows an assembly comprising the dilution spacer 230 of the present invention in combination with a metered-dose inhaler 100 as shown in Figure 1. As described above, the metered-dose inhaler 100 comprises a canister 102, a metering valve 104 and an actuator 106, with the canister 102 received in the body 114 of the actuator 106 such that the valve stem 112 of the metering valve 104, which is sealed to the canister 102, is received by the actuator nozzle 118, which is configured to generate a metered-dose inhaler plume 120 (Figure 10B) from the medication 108 in the canister 102. As described above, the actuator inlet 242 is configured to securely releasably interengage the actuator mouthpiece 116, and the metered-dose inhaler 100 is coupled to the dilution spacer 230 by interengagement of the actuator mouthpiece 116 and the actuator inlet 242, with the body 114 of the actuator 104 substantially flush with the edge of the enclosure 232. In this position, the actuator nozzle 118 will direct the plume 120 into the dilution chamber 234 through the actuator mouthpiece 116 and the actuator inlet 242; as noted above the actuator nozzle 118 will direct the plume 120 (Figure 10B) at an oblique angle to the valve stem 112.

Operation of the assembly comprising the dilution spacer 230 of the present invention and metered-dose inhaler 100 will now be described with reference to Figure 10B. In use, a user would seal his or her mouth around the outlet 250 (e.g. seal his or her lips around the dilution spacer mouthpiece 252) and inhale through his or her mouth. This inhalation creates suction through the outlet 250 from outside the enclosure 232 which, because the ambient air inlet 248 is positioned opposite the outlet 250, draws ambient air into the enclosure 232 through the ambient air inlet 248 to generate a fast airflow path, denoted by arrow FAF, from the ambient air inlet 248 through the dilution chamber 234 and out of the outlet 250 into the user's mouth. The geometry of the dilution chamber 234 formed by the enclosure 232 is such that the inhalation also generates an air recirculation zone, denoted by arrows ARZ, where some of the air drawn into the enclosure 232 bleeds off from the fast airflow path FAP and recirculates.

While inhaling, the user would push the canister 102 toward the actuator nozzle 118 so as to move the valve stem 112 (relative to the canister 102) into the dispensing position and release the metered quantity of medication 108 into the actuator nozzle 118 to generate the plume 120, as shown in Figure 10B. The actuator inlet 242 is positioned, relative to the ambient air inlet 248 and the outlet 250, so that when the plume 120 enters the dilution chamber 234 through the actuator inlet 242, the plume 120 intersects the fast airflow path FAF. As a result, airflow (generated by the user's inhalation) along the fast airflow path FAP entrains and redirects at least a portion 124 of the plume 120 toward the outlet 250 so that it will be inhaled by the user. The dilution chamber 234 is configured and the actuator inlet 242 is positioned and configured, relative to the ambient air inlet 248 and the outlet 250, so that the plume 120 entering the dilution chamber 234 through the actuator inlet 242 is configured to intersect the fast airflow path FAP. The plume 120 is configured to have adequate distance from the fast airflow path FAP to enable the plume 120 to spread before the plume 120 intersects the fast airflow path FAP and airflow along the fast airflow path FAP is configured to entrain and redirect at least a portion of the plume 120 toward the outlet 250.

According to the present invention, the extremely fast aerosol plume 120 from the actuator nozzle 118 has adequate distance to disperse and develop before it then encounters a fast air jet of comparable velocity, namely the airflow along the fast airflow path FAP. This competitive air speed efficiently entrains the aerosol particles 126 that are small enough to penetrate deeply into the lungs, leaving only large particles (which would have ended up in the user's throat with unaided MDI use) to impact the edge wall 238 opposite the actuator nozzle 118. However, because the fast airflow path FAP is narrow and makes up a minority of the volume of the dilution chamber 234, the volumetric flow rate is low. This has the advantage of prolonging and slowing the inhalation cycle and extending the time over which the aerosol particles are delivered. More particularly, because some of the air drawn into the enclosure 232 bleeds off of the fast airflow path FAP and recirculates in the air recirculation zone ARZ with small aerosol particles 126 entrained therein, the effective inhalation cycle is prolonged. As a user continues to inhale after completion of the discharge from the actuator nozzle 118, he or she can continue to inhale dispersed medication as the medicated air from the air recirculation zone ARZ rejoins the fast airflow path FAP. The relative dimensions of the dilution chamber 234 and the position and orientation of the fast airflow path FAP between the ambient air inlet 248 and the outlet 250 facilitates high velocity flow to effectively entrain small aerosol particles 126, but low volumetric flow rate. This is of particular importance for the effective administration of aerosol medication, because it significantly reduces the impact of the timing of MDI actuation relative to the inhalation cycle. As long as MDI actuation occurs after commencement of inhalation and during the early part of a long and slow deep inhalation, the aerosol particles 126 recirculate until depleted or until the user has fully expanded their lungs and cannot further inhale. An additional benefit is that a given dose of aerosol medication is dispersed within a larger volume of inhaled air (as compared to direct inhalation from the MDI or from a conventional holding chamber or spacer), which can increase tolerability of the medication.

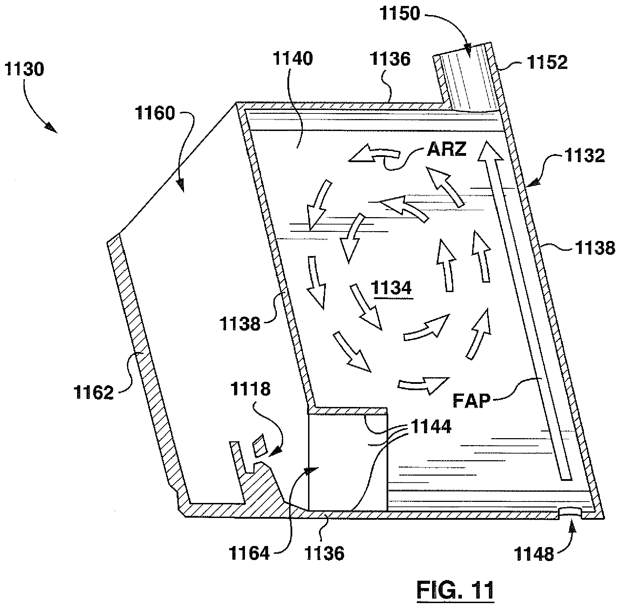

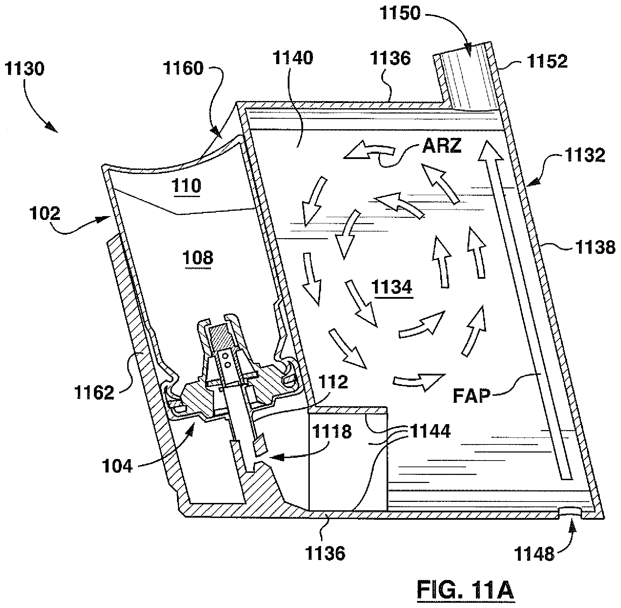

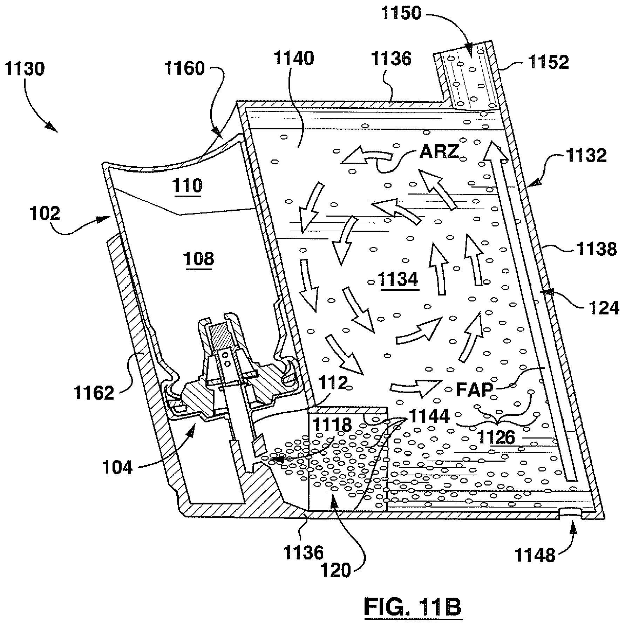

Reference is now made to Figures 11 to 11B, in which an illustrative metered-dose inhaler actuator according to the present invention is indicated generally by reference 1130. The metered-dose inhaler actuator 1130 shown in Figures 11A and 11B is an apparatus which integrates a metered-dose inhaler actuator and a dilution chamber, and may be considered as a combination of the actuator 106 and the dilution spacer 230 into a single integral unit. As such, like reference numerals denote like features, except with the prefix "11" instead of "2".

The metered-dose inhaler actuator 1130 comprises an enclosure 1132 formed by a pair of opposed outwardly curved end walls 1136, a pair of opposed generally planar edge walls 1138 and a pair of opposed generally planar side walls 1140. While the illustrative enclosure 1132 is generally parallelepipedic, it may have other suitable shapes as well. The enclosure 1132 defines a dilution chamber 1134.

An ambient air inlet 1148 is formed through one of the end walls 1136 and an outlet 1 ISO is formed through the other one of the end walls 1136 opposite the ambient air inlet. The ambient air inlet 1148 and the outlet 1150 are in fluid communication with the dilution chamber 1134. In the illustrated embodiment, the outlet 1150 comprises an outwardly projecting dilution mouthpiece 1152; in other embodiments the mouthpiece may have different shapes or be omitted entirely.

The metered-dose inhaler actuator 1130 further comprises a receptacle 1160 having an actuator nozzle 1118 and configured to securely releasably receive a metered-dose inhaler canister 102 so that a valve stem 112 of a metering valve 104 sealed to the canister 102 is received by the actuator nozzle 1118, as shown in Figure 11B. In the illustrated embodiment one of the edge walls 1138 and the end wall 1136 through which the ambient air inlet 1148 is formed cooperates with an encircling wall 1162 to form the receptacle 1160 although other configurations are also contemplated. The actuator nozzle 1118 is in fluid communication with the dilution chamber 1134 through an opening or gap 1164 (Figure 11) between the edge wall 1138 and the end wall 1136 that form the receptacle 1160 in conjunction with guide walls 1144; in other embodiments the edge wall may extend fully to the end wall and the actuator nozzle may be in fluid communication with the dilution chamber through an aperture in the edge wall.

The actuator nozzle 1118 is configured to generate a plume 120 (Figure 11B) from the medication 108 in the canister 102 and direct the plume 120 into the dilution chamber 1134 through the gap 1164 between the edge wail 1138 and the end wall 1136; in the illustrated embodiment the actuator nozzle 1118 will direct the plume 120 (Figure 11B) at an oblique angle to the valve stem 112.

Operation of the metered-dose inhaler actuator 1130 of the present invention shown in Figures 11A and 11B is similar to operation of the assembly comprising the dilution spacer 230 and metered-dose inhaler 100 described with reference to Figures 10A and 10B. A user would seal his or her mouth around the outlet 1150 and inhale, creating suction through the outlet 1150 from outside the enclosure 1132. Since the ambient air inlet 1148 is positioned opposite the outlet 1150, this suction draws ambient air into the enclosure 1132 through the ambient air inlet 1148, which generates a fast airflow path FAP from the ambient air inlet 1148 through the dilution chamber 1134 and out of the outlet 1150 into the user's mouth. During inhalation, the user actuates the metering valve 104 to release the metered quantity of medication 108 into the actuator nozzle 1118 to generate the plume 120, as shown in Figure 11B. The position of the opening or gap 1164 (Figure 11) relative to the ambient air inlet 1148 and the outlet 1150 is such that when the plume 120 enters the dilution chamber 1134 through the opening or gap 1164 (Figure 11), the plume 120 intersects the fast airflow path FAP. The inhalation airflow along the fast airflow path FAP then entrains and redirects at least a portion 124 of the plume 120toward the outlet 1150 to be inhaled by the user. The geometry of the dilution chamber 1134 formed by the enclosure 1132 results in the inhalation generating an air recirculation zone ARZ into which some of the air drawn into the enclosure 1132 bleeds off from the fast airflow path FAP and recirculates. As a user continues to inhale after completion of the discharge from the actuator nozzle 1118, he or she can continue to inhale dispersed medication as the medicated air from the air recirculation zone ARZ rejoins the fast airflow path FAP and moves toward the outlet 1150.

Without being limited by theory, and without promising any particular utility, it is believed that the technology disclosed herein enables a slowing of the ordinarily high inhalation air volumetric flow rate with coincident slowing, dispersion and entrainment of the medication in the plume 120, reducing the timing sensitivity of actuation of the metering valve 104. Instead of having to time inhalation and actuation to be substantially coincident (to achieve inhalation instead of having the medication impact the inside of the mouth or settle inside of a conventional holding chamber or spacer), the user can begin a slow, deep inhalation and then actuate the metering valve 104 while continuing to inhale. Thus, again without being limited by theory, and without promising any particular utility, it is believed that the technology disclosed herein enables a more prolonged inhalation cycle, which facilitates deep lung penetration of the medication while reducing oropharyngeal deposition and dose losses by impaction on the holding chamber or spacer walls, all while maintaining a compact and discreet overall geometry. In the illustrated embodiment, die desired geometry is achieved by turning the aerosol plume 120 by entrainment to flow substantially parallel to the longitudinal axis of the canister 102. Thus, in the illustrated embodiments, the plume 120 entering the dilution chamber 234, 1134 intersects the fast airflow path FAP non-parallel (and also non-perpendicular) thereto and the airflow along the fast airflow path FAP redirects at least a portion of the metered-dose inhaler plume 120 by about 103.5 degrees. In this context, the direction of the plume 120 is defined by a notional centroid line of the plume.

While the illustrated embodiments redirect (at least part of) the plume 120 by about - 103.5 degrees, redirection by smaller or larger angles, up to 180 degrees, is also contemplated. In the case of a redirection by 180 degrees (i.e. a reversal of direction), the airflow would surround and move past the canister, but in the opposite direction from that of the plume. In each case, the airflow generated by inhalation is not flowing in the same direction as the plume leaving the actuator nozzle, and in a preferred embodiments the airflow is substantially parallel to the longitudinal axis of the canister.

Certain illustrative embodiments have been described by way of example. It will be apparent to persons skilled in the art that a number of variations and modifications can be made without departing from the scope of the invention as defined in the claims.

## Claims

1. A dilution spacer (230) for a metered-dose inhaler (100), comprising:
an enclosure (232) defining a dilution chamber (234);
an actuator inlet (242) configured to securely releasably interengage a metered-dose inhaler actuator mouthpiece (116);
an ambient air inlet (248); and
an outlet (250);
wherein:
each of the actuator inlet (242), the ambient air inlet (248) and the outlet (250) are in fluid communication with the dilution chamber (234);
the ambient air inlet (248) is positioned opposite the outlet (250) whereby suction through the outlet from outside the enclosure (232) draws ambient air into the enclosure (232) through the ambient air inlet (248) to generate a fast air jet along a fast airflow path (FAP) from the ambient air inlet (248) through the dilution chamber (234) and out of the outlet (250); and
the actuator inlet (242) is positioned relative to the ambient air inlet (248) and the outlet (250) so that a metered-dose inhaler plume (120) entering the dilution chamber (234) through the actuator inlet (242) intersects the fast air jet along the fast airflow path (FAP) and has adequate distance to disperse and
develop before it encounters the fast air jet;
wherein the metered-dose inhaler plume (120) is of comparable velocity to the fast air jet, whereby the fast air jet along the fast airflow path (FAP) entrains and redirects toward the outlet small aerosol particles (126) of the metered-dose inhaler plume (120) that are small enough to penetrate deeply into the lungs, leaving only large particles to impact an edge wall (238) opposite the actuator nozzle (118);
**characterized in that**:
the fast airflow path (FAP) is narrow and makes up a minority of the volume of the dilution chamber (234) and has a low volumetric flow rate to prolong and slow inhalation and extend the time over which the aerosol particles are delivered; and
the geometry of the dilution chamber (234) formed by the enclosure (232) is such that the suction also generates an air recirculation zone (ARZ) where some of the air drawn into the enclosure bleeds off from the fast airflow path (FAP) and recirculates in the air recirculation zone (ARZ) with the small aerosol (126) particles entrained therein, and then rejoins the fast airflow path (FAP).

2. An assembly comprising:
the dilution spacer (230) of claim 1; and
a metered-dose inhaler actuator (106) whose actuator mouthpiece (116) is securely releasably interengaged in the actuator inlet (242).

3. The assembly of claim 2, wherein the actuator mouthpiece (116) is friction fit in the actuator inlet (242).

4. The assembly of claim 2, wherein the actuator mouthpiece (116) is interference fit in the actuator inlet (242).

5. The assembly of claim 2, wherein:
a metered-dose inhaler canister (102) is received in a body (114) of the metered-dose inhaler actuator (106);
a valve stem (112) of a metering valve (104) sealed to the metered-dose inhaler canister (102) is received by an actuator nozzle (118) of the metered-dose inhaler actuator (106); and
the actuator nozzle (118) is configured to:
generate a metered-dose inhaler plume (120) from contents (108) of the metered-dose inhaler canister (102); and
direct the metered-dose inhaler plume (120) into the dilution chamber (234) through the actuator mouthpiece (116) and the actuator inlet (242).

6. The assembly of claim 5, wherein the actuator nozzle (118) is configured to direct the metered-dose inhaler plume (120) at an oblique angle to the valve stem (112).

7. The assembly of claim 6, wherein:
the enclosure (232) is generally parallelepipedic;
the actuator mouthpiece (116) of the metered-dose inhaler actuator (106) is at an oblique angle to the body (114) of the metered-dose inhaler actuator (106); and
the body (114) of the metered-dose inhaler actuator (106) is substantially flush with an edge (238) of the enclosure (232) in which the actuator inlet (242) is formed.

8. The assembly of claim 5, wherein the metered-dose inhaler plume (120) entering the dilution chamber (234) through the actuator inlet (242) intersects the fast airflow path (FAP) non-parallel thereto.

9. The dilution spacer (230) of claim 1, wherein the fast air jet redirects at least a portion of the metered-dose inhaler plume (120) by about 103.5 degrees.

10. The dilution spacer (230) of claim 1, wherein the enclosure (232) is generally parallelepipedic.

11. The dilution spacer (230) of claim 1, wherein the outlet (250) comprises a dilution spacer mouthpiece (252) projecting outwardly from the enclosure (232).

12. A metered-dose inhaler actuator (1130), comprising:
an enclosure (1132) defining a dilution chamber (1134);
a receptacle (1160) having an actuator nozzle (1118) and configured to receive a metered-dose inhaler canister (102) so that a valve stem (112) of a metering valve (104) sealed to the metered-dose inhaler canister (102) is received by the actuator nozzle (1118);
an ambient air inlet (1148);
an outlet (1150);
wherein:
each of the actuator nozzle (1118), the ambient air inlet (1148) and the outlet (1150) are in fluid communication with the dilution chamber (1134);
the ambient air inlet (1148) is positioned opposite the outlet (1150) whereby suction through the outlet (1150) from outside the enclosure (1132) draws ambient air into the enclosure (1132) through the ambient air inlet (1148) to generate a fast air jet along a fast airflow path (FAP) from the ambient air inlet (1148) through the dilution chamber (1134) and out of the outlet (1150);
the actuator nozzle (1118) is configured to:
generate a metered-dose inhaler plume (120) from contents (108) of the metered-dose inhaler canister (102); and
direct the metered-dose inhaler plume (120) into the dilution chamber (1134); and
the actuator nozzle (1118) is positioned relative to the ambient air inlet (1148) and the outlet (1150) so that the metered-dose inhaler plume (120) intersects the fast air jet along the fast airflow path (FAP) and has adequate distance to disperse and develop before it encounters the fast air jet;
wherein the metered-dose inhaler plume (120) is of comparable velocity to the fast air jet, whereby the fast air jet along the fast airflow path (FAP) entrains and redirects oward the outlet small aerosol particles (126) of the metered-dose inhaler plume (120) that are small enough to penetrate deeply into the lungs, leaving only large particles to impact an edge wall (238) opposite the actuator nozzle (118);
**characterized in that**:
the fast airflow path (FAP) is narrow and makes up a minority of the volume of the dilution chamber (1134) and has a low volumetric flow rate to prolong and slow inhalation and extend the time over which the aerosol particles are delivered; and
the geometry of the dilution chamber (1134) formed by the enclosure (1132) is such that the suction also generates an air recirculation zone (ARZ) where some of the air drawn into the enclosure (1132) bleeds off from the fast airflow path (FAP) and recirculates in the air recirculation zone (ARZ) with the small aerosol particles (126) entrained therein, and then rejoins the fast airflow path (FAP).

13. The metered-dose inhaler actuator (1130) of claim 12, wherein the outlet (1150) comprises a mouthpiece (1152) projecting outwardly from the enclosure (1132).

14. An assembly comprising:
the metered-dose inhaler actuator (1130) of claim 12; and
a metered-dose inhaler canister (102) received within the receptacle (1160) with the valve stem (112) of the metering valve (104) of the metered-dose inhaler canister (102) received by the actuator nozzle (1118).

15. The assembly of claim 14, wherein the actuator nozzle (1118) is configured to direct the metered-dose inhaler plume (120) at an oblique angle to the valve stem (112).

16. The assembly of claim 14, wherein the metered-dose inhaler plume (120) entering the dilution chamber (1134) through the actuator nozzle (1118) intersects the fast airflow path (FAP) non-parallel thereto.

17. The metered-dose inhaler actuator (1130) of claim 12, wherein the fast air jet redirects at least a portion of the metered-dose inhaler plume (120) by about 103.5 degrees.

## Patentansprüche

1. Verdünnungsdistanzstück (230) für ein Dosier-Aerosol (100), umfassend:
ein Gehäuse (232), das eine Verdünnungskammer (234) definiert;
einen Aktuatoreinlass (242), der dazu ausgestaltet ist, ein Dosier-Aerosolaktuatormundstück (116) sicher lösbar in Eingriff zu nehmen;
einen Umgebungslufteinlass (248); und
einen Auslass (250);
wobei:
jeder des Aktuatoreinlasses (242), des Umgebungslufteinlasses (248) und des Auslasses (250) in Fluidverbindung mit der Verdünnungskammer (234) stehen;
der Umgebungslufteinlass (248) gegenüber dem Auslass (250) positioniert ist, wobei ein Ansaugen durch den Auslass von außerhalb des Gehäuses (232) Umgebungsluft durch den Umgebungslufteinlass (248) in das Gehäuse (232) zieht, um einen schnellen Luftstrahl entlang eines schnellen Luftströmungswegs (FAP) von dem Umgebungslufteinlass (248) durch die Verdünnungskammer (234) und aus dem Auslass (250) zu erzeugen; und
der Aktuatoreinlass (242) relativ zu dem Umgebungslufteinlass (248) und dem Auslass (250) derart positioniert ist, dass ein durch den Aktuatoreinlass (242) in die Verdünnungskammer (234) eintretender Dosier-Aerosolnebel (120) den schnellen Luftstrahl entlang des schnellen Luftströmungswegs (FAP) schneidet und eine ausreichende Distanz zum Verteilen und Entwickeln hat, bevor er auf den schnellen Luftstrahl trifft;
der Dosier-Aerosolnebel (120) eine vergleichbare Geschwindigkeit wie der schnelle Luftstrahl aufweist, wobei der schnelle Luftstrahl entlang des schnellen Luftströmungswegs (FAP) kleine Aerosolteilchen (126) des Dosier-Aerosolnebels (120), die klein genug sind, um tief in die Lunge vorzudringen, mitreißt und zum Auslass hin umleitet, so dass nur große Teilchen verbleiben, die auf eine Randwand (238) gegenüber der Aktuatordüse (118) aufprallen;
**dadurch gekennzeichnet, dass**:
der schnelle Luftströmungsweg (FAP) schmal ist und einen kleinen Teil des Volumens der Verdünnungskammer (234) ausmacht und eine niedrige Volumenströmungsrate aufweist, um eine Inhalation zu prolongieren und zu verlangsamen und die Zeit zu verlängern, über welche die Aerosolteilchen abgegeben werden; und
die Geometrie der durch das Gehäuse (232) gebildeten Verdünnungskammer (234) derart ist, dass das Ansaugen auch eine Luftrezirkulationszone (ARZ) erzeugt, in der ein Teil der in das Gehäuse gezogenen Luft aus dem schnellen Luftströmungsweg (FAP) entlüftet und in der Luftrezirkulationszone (ARZ) mit den darin mitgerissenen kleinen Aerosol(126)-Teilchen rezirkuliert und sich anschließend wieder mit dem schnellen Luftströmungsweg (FAP) vereint.

2. Anordnung, umfassend:
das Verdünnungsdistanzstück (230) nach Anspruch 1; und
einen Dosier-Aerosolaktuator (106), dessen Aktuatormundstück (116) sicher lösbar in dem Aktuatoreinlass (242) in Eingriff steht.

3. Anordnung nach Anspruch 2, wobei das Aktuatormundstück (116) in Reibpassung in dem Aktuatoreinlass (242) steht.

4. Anordnung nach Anspruch 2, wobei das Aktuatormundstück (116) in Übermaßpassung in dem Aktuatoreinlass (242) steht.

5. Anordnung nach Anspruch 2, wobei:
ein Dosier-Aerosolbehälter (102) in einem Körper (114) des Dosier-Aerosolaktuators (106) aufgenommen ist;
ein Ventilschaft (112) eines Dosierventils (104), das an den Dosier-Aerosolbehälter (102) gesiegelt ist, durch eine Aktuatordüse (118) des Dosier-Aerosolaktuators (106) aufgenommen ist; und
die Aktuatordüse (118) zu Folgendem ausgestaltet ist:
Erzeugen eines Dosier-Aerosolnebels (120) aus Inhaltsstoffen (108) des Dosier-Aerosolbehälters (102); und
Leiten des Dosier-Aerosolnebels (120) durch das Aktuatormundstück (116) und den Aktuatoreinlass (242) in die Verdünnungskammer (234).

6. Anordnung nach Anspruch 5, wobei die Aktuatordüse (118) dazu ausgestaltet ist, den Dosier-Aerosolnebel (120) in einem schiefen Winkel zu dem Ventilschaft (112) zu leiten.

7. Anordnung nach Anspruch 6, wobei:
das Gehäuse (232) im Allgemeinen parallelepipedförmig ist;
das Aktuatormundstück (116) des Dosier-Aerosolaktuators (106) in einem schiefen Winkel zu dem Körper (114) des Dosier-Aerosolaktuators (106) steht; und
der Körper (114) des Dosier-Aerosolaktuators (106) im Wesentlichen bündig mit einem Rand (238) des Gehäuse (232) ist, in welchem der Aktuatoreinlass (242) ausgebildet ist.

8. Anordnung nach Anspruch 5, wobei der durch den Aktuatoreinlass (242) in die Verdünnungskammer (234) eintretende Dosier-Aerosolnebel (120) den schnellen Luftströmungsweg (FAP) nicht-parallel dazu schneidet.

9. Verdünnungsdistanzstück (230) nach Anspruch 1, wobei der schnelle Luftstrahl zumindest einen Teil des Dosier-Aerosolnebels (120) um etwa 103,5 Grad umleitet.

10. Verdünnungsdistanzstück (230) nach Anspruch 1, wobei das Gehäuse (232) im Allgemeinen parallelepipedförmig ist.

11. Verdünnungsdistanzstück (230) nach Anspruch 1, wobei der Auslass (250) ein Verdünnungsdistanzstückmundstück (252) aufweist, das von dem Gehäuse (232) nach außen hervorsteht.

12. Dosier-Aerosolaktuator (1130), umfassend:
ein Gehäuse (1132), das eine Verdünnungskammer (1134) definiert;
eine Aufnahme (1160), die eine Aktuatordüse (1118) aufweist und dazu ausgestaltet ist, einen Dosier-Aerosolbehälter (102) derart aufzunehmen, dass ein Ventilschaft (112) eines Dosierventils (104), das an den Dosier-Aerosolbehälter (102) gesiegelt ist, durch die Aktuatordüse (1118) aufgenommen ist;
einen Umgebungslufteinlass (1148);
einen Auslass (1150);
wobei:
jede der Aktuatordüse (1118), des Umgebungslufteinlasses (1148) und des Auslasses (1150) in Fluidverbindung mit der Verdünnungskammer (1134) stehen;
der Umgebungslufteinlass (1148) gegenüber dem Auslass (1150) positioniert ist, wobei ein Ansaugen durch den Auslass (1150) von außerhalb des Gehäuses (1132) Umgebungsluft in das Gehäuse (1132) durch den Umgebungslufteinlass (1148) zieht, um einen schnellen Luftstrahl entlang eines schnellen Luftströmungswegs (FAP) von dem Umgebungslufteinlass (1148) durch die Verdünnungskammer (1134) und aus dem Auslass (1150) zu erzeugen;
die Aktuatordüse (1118) zu Folgendem ausgestaltet ist:
Erzeugen eines Dosier-Aerosolnebels (120) aus Inhaltsstoffen (108) des Dosier-Aerosolbehälters (102); und
Leiten des Dosier-Aerosolnebels (120) in die Verdünnungskammer (1134); und
wobei die Aktuatordüse (1118) relativ zu dem Umgebungslufteinlass (1148) und dem Auslass (1150) derart positioniert ist, dass der Dosier-Aerosolnebel (120) den schnellen Luftstrahl entlang des schnellen Luftströmungswegs (FAP) schneidet und eine ausreichende Distanz zum Verteilen und Entwickeln hat, bevor er auf den schnellen Luftstrahl trifft;
wobei der Dosier-Aerosolnebel (120) eine vergleichbare Geschwindigkeit wie der schnelle Luftstrahl aufweist,
wobei der schnelle Luftstrahl entlang des schnellen Luftströmungswegs (FAP) kleine Aerosolteilchen (126) des Dosier-Aerosolnebels (120), die klein genug sind, um tief in die Lunge vorzudringen, mitreißt und zum Auslass hin umleitet, so dass nur große Teilchen verbleiben, die auf eine Randwand (238) gegenüber der Aktuatordüse (118) aufprallen;
**dadurch gekennzeichnet, dass**:
der schnelle Luftströmungsweg (FAP) schmal ist und einen kleinen Teil des Volumens der Verdünnungskammer (1134) ausmacht und eine niedrige Volumenströmungsrate aufweist, um eine Inhalation zu prolongieren und zu verlangsamen und die Zeit zu verlängern, über welche Aerosolteilchen abgegeben werden; und
die Geometrie der durch das Gehäuse (1132) gebildeten Verdünnungskammer (1134) derart ist, dass das Ansaugen auch eine Luftrezirkulationszone (ARZ) erzeugt, in der ein Teil der in das Gehäuse (1132) gezogenen Luft aus dem schnellen Luftströmungsweg (FAP) entlüftet und in der Luftrezirkulationszone (ARZ) mit den darin mitgerissenen kleinen Aerosolteilchen (126) rezirkuliert und sich anschließend wieder mit dem schnellen Luftströmungsweg (FAP) vereint.

13. Dosier-Aerosolaktuator (1130) nach Anspruch 12, wobei der Auslass (1150) ein Mundstück (1152) aufweist, das von dem Gehäuse (1132) nach außen hervorsteht.

14. Anordnung, umfassend:
den Dosier-Aerosolaktuator (1130) nach Anspruch 12; und
einen in der Aufnahme (1160) aufgenommenen Dosier-Aerosolbehälter (102), wobei der Ventilschaft (112) des Dosierventils (104) des Dosier-Aerosolbehälters (102) durch die Aktuatordüse (1118) aufgenommen ist.

15. Anordnung nach Anspruch 14, wobei die Aktuatordüse (1118) dazu ausgestaltet ist, den Dosier-Aerosolnebel (120) in einem schiefen Winkel zu dem Ventilschaft (112) zu leiten.

16. Anordnung nach Anspruch 14, wobei der durch die Aktuatordüse (1118) in die Verdünnungskammer (1134) eintretende Dosier-Aerosolnebel (120) den schnellen Luftströmungsweg (FAP) nicht-parallel dazu schneidet.

17. Dosier-Aerosolaktuator (1130) nach Anspruch 12, wobei der schnelle Luftstrahl zumindest einen Teil des Dosier-Aerosolnebels (120) um etwa 103,5 Grad umleitet.

## Revendications

1. Espaceur de dilution (230) pour un inhalateur-doseur (100), comprenant :
une enceinte (232) définissant une chambre de dilution (234) ;
une entrée d'actionneur (242) conçue pour venir en prise de manière sécurisée et libérable avec un embout d'actionneur d'inhalateur-doseur (116) ;
une entrée d'air ambiant (248) ; et
une sortie (250) ;
chacune de l'entrée d'actionneur (242), de l'entrée d'air ambiant (248) et de la sortie (250) étant en communication fluidique avec la chambre de dilution (234) ;
l'entrée d'air ambiant (248) étant positionnée en face de la sortie (250) moyennant quoi l'aspiration à travers la sortie depuis l'extérieur de l'enceinte (232) attire l'air ambiant dans l'enceinte (232) à travers l'entrée d'air ambiant (248) pour générer un jet d'air rapide le long d'un trajet d'écoulement d'air rapide (FAP) depuis l'entrée d'air ambiant (248) à travers la chambre de dilution (234) et hors de la sortie (250) ; et
l'entrée d'actionneur (242) étant positionnée par rapport à l'entrée d'air ambiant (248) et à la sortie (250) de sorte qu'un panache d'inhalateur-doseur (120) entrant dans la chambre de dilution (234) à travers l'entrée d'actionneur (242) coupe le jet d'air rapide le long du trajet d'écoulement d'air rapide (FAP) et dispose d'une distance suffisante pour se disperser et se développer avant de rencontrer le jet d'air rapide ;
le panache d'inhalateur-doseur (120) ayant une vitesse comparable à celle du jet d'air rapide, moyennant quoi le jet d'air rapide le long du trajet d'écoulement d'air rapide (FAP) entraîne et redirige vers la sortie les petites particules d'aérosol (126) du panache d'inhalateur-doseur (120) qui sont suffisamment petites pour pénétrer profondément dans les poumons, ne laissant que les grosses particules frapper une paroi de bord (238) à l'opposé de la buse d'actionneur (118) ;
**caractérisé en ce que** :
le trajet d'écoulement d'air rapide (FAP) est étroit et représente une minorité du volume de la chambre de dilution (234) et a un faible débit volumétrique afin de prolonger et de ralentir l'inhalation et d'allonger le temps pendant lequel les particules d'aérosol sont administrées ; et
la géométrie de la chambre de dilution (234) formée par l'enceinte (232) est telle que l'aspiration génère également une zone de recirculation d'air (ARZ) dans laquelle une partie de l'air aspiré dans l'enceinte s'échappe du trajet d'écoulement d'air rapide (FAP) et recircule dans la zone de recirculation d'air (ARZ) avec les petites particules d'aérosol (126) qui y sont entraînées, puis rejoint le trajet d'écoulement d'air rapide (FAP).

2. Ensemble, comprenant :
l'espaceur de dilution (230) selon la revendication 1 ; et
un actionneur d'inhalateur-doseur (106) dont l'embout (116) vient en prise de manière sécurisée et libérable avec l'entrée d'actionneur (242).

3. Ensemble selon la revendication 2, l'embout d'actionneur (116) étant ajusté par friction dans l'entrée d'actionneur (242).

4. Ensemble selon la revendication 2, l'embout d'actionneur (116) étant en ajustement serré dans l'entrée d'actionneur (242).

5. Ensemble selon la revendication 2,
une cartouche d'inhalateur-doseur (102) étant reçue dans un corps (114) de l'actionneur d'inhalateur-doseur (106) ;
une tige de soupape (112) d'une soupape de dosage (104) scellée à la cartouche d'inhalateur-doseur (102) étant reçue par une buse d'actionneur (118) de l'actionneur d'inhalateur-doseur (106) ; et
la buse d'actionneur (118) étant conçue pour :
générer un panache d'inhalateur-doseur (120) à partir du contenu (108) de la cartouche d'inhalateur-doseur (102) ; et
diriger le panache d'inhalateur-doseur (120) dans la chambre de dilution (234) à travers l'embout d'actionneur (116) et l'entrée d'actionneur (242).

6. Ensemble selon la revendication 5, la buse d'actionneur (118) étant conçue pour diriger le panache d'inhalateur-doseur (120) à un angle oblique par rapport à la tige de soupape (112).

7. Ensemble selon la revendication 6,
l'enceinte (232) étant généralement parallélépipédique ;
l'embout d'actionneur (116) de l'inhalateur-doseur (106) formant un angle oblique par rapport au corps (114) de l'actionneur d'inhalateur-doseur (106) ; et
le corps (114) de l'actionneur d'inhalateur-doseur (106) étant sensiblement au même niveau qu'un bord (238) de l'enceinte (232) dans laquelle l'entrée d'actionneur (242) est formée.

8. Ensemble selon la revendication 5, le panache d'inhalateur-doseur (120) entrant dans la chambre de dilution (234) à travers l'entrée d'actionneur (242) coupant le trajet d'écoulement d'air rapide (FAP) de manière non parallèle à celle-ci.

9. Espaceur de dilution (230) selon la revendication 1, le jet d'air rapide redirigeant au moins une partie du panache d'inhalateur-doseur (120) d'environ 103,5 degrés.

10. Espaceur de dilution (230) selon la revendication 1, l'enceinte (232) étant généralement parallélépipédique.

11. Espaceur de dilution (230) selon la revendication 1, la sortie (250) comprenant un embout d'espaceur de dilution (252) faisant saillie vers l'extérieur depuis l'enceinte (232).

12. Actionneur d'inhalateur-doseur (1130), comprenant :
une enceinte (1132) définissant une chambre de dilution (1134) ;
un récipient (1160) comportant une buse d'actionneur (1118) et conçu pour recevoir une cartouche d'inhalateur-doseur (102) de sorte qu'une tige de soupape (112) d'une soupape de dosage (104) scellée à la cartouche d'inhalateur-doseur (102) soit reçue par la buse d'actionneur (1118) ;
une entrée d'air ambiant (1148) ;
une sortie (1150) ;
chacune de la buse d'actionneur (1118), de l'entrée d'air ambiant (1148) et de la sortie (1150) étant en communication fluidique avec la chambre de dilution (1134) ;
l'entrée d'air ambiant (1148) étant positionnée en face de la sortie (1150) de sorte que l'aspiration à travers la sortie (1150) depuis l'extérieur de l'enceinte (1132) attire l'air ambiant dans l'enceinte (1132) à travers l'entrée d'air ambiant (1148) pour générer un jet d'air rapide le long d'un trajet d'écoulement d'air rapide (FAP) depuis l'entrée d'air ambiant (1148) à travers la chambre de dilution (1134) et hors de la sortie (1150) ;
la buse d'actionneur (1118) étant conçue pour :
générer un panache d'inhalateur-doseur (120) à partir du contenu (108) de la cartouche d'inhalateur-doseur (102) ; et
diriger le panache d'inhalateur-doseur (120) dans la chambre de dilution (1134) ; et
la buse d'actionneur (1118) étant positionnée par rapport à l'entrée d'air ambiant (1148) et à la sortie (1150) de sorte que le panache d'inhalateur-doseur (120) coupe le jet d'air rapide le long du trajet d'écoulement d'air rapide (FAP) et dispose d'une distance suffisante pour se disperser et se développer avant de rencontrer le jet d'air rapide ;
le panache d'inhalateur-doseur (120) ayant une vitesse comparable à celle du jet d'air rapide, moyennant quoi le jet d'air rapide le long du trajet d'écoulement d'air rapide (FAP) entraîne et redirige vers la sortie les petites particules d'aérosol (126) du panache d'inhalateur-doseur (120) qui sont suffisamment petites pour pénétrer profondément dans les poumons, ne laissant que les grosses particules frapper une paroi de bord (238) à l'opposé de la buse d'actionneur (118) ;
**caractérisé en ce que** :
le trajet d'écoulement d'air rapide (FAP) est étroit et représente une minorité du volume de la chambre de dilution (1134) et a un faible débit volumétrique afin de prolonger et de ralentir l'inhalation et d'allonger le temps pendant lequel les particules d'aérosol sont administrées ; et
la géométrie de la chambre de dilution (1134) formée par l'enceinte (1132) est telle que l'aspiration génère également une zone de recirculation d'air (ARZ) dans laquelle une partie de l'air aspiré dans l'enceinte (1132) s'échappe du trajet d'écoulement d'air rapide (FAP) et recircule dans la zone de recirculation d'air (ARZ) avec les petites particules d'aérosol (126) qui y sont entraînées, puis rejoint le trajet d'écoulement d'air rapide (FAP).

13. Actionneur d'inhalateur-doseur (1130) selon la revendication 12, la sortie (1150) comprenant un embout (1152) faisant saillie vers l'extérieur de l'enceinte (1132) .

14. Ensemble, comprenant :
l'actionneur d'inhalateur-doseur (1130) selon la revendication 12 ; et
une cartouche d'inhalateur-doseur (102) reçue à l'intérieur du récipient (1160), la tige de soupape (112) de la soupape de dosage (104) de la cartouche d'inhalateur-doseur (102) étant reçue par la buse d'actionneur (1118).

15. Ensemble selon la revendication 14, la buse d'actionneur (1118) étant conçue pour diriger le panache d'inhalateur-doseur (120) à un angle oblique par rapport à la tige de soupape (112).

16. Ensemble selon la revendication 14, le panache d'inhalateur-doseur (120) entrant dans la chambre de dilution (1134) à travers la buse d'actionneur (1118) coupant le trajet d'écoulement rapide d'air (FAP) de manière non parallèle à celle-ci.

17. Actionneur d'inhalateur-doseur (1130) selon la revendication 12, le jet d'air rapide redirigeant au moins une partie du panache d'inhalateur-doseur (120) d'environ 103,5 degrés.
